# EUROPEAN PATENT APPLICATION

(11) **EP 1 932 425 A2**
(43) Date of publication of application: **18.06.2008**
(21) Application number: 07123001.5
(22) Date of filing: 12.12.2007
(51) Int. Cl.: A01N 59/00, A01N 41/06, A01N 37/44, A01N 25/12, A01P 1/00, A61L 2/16, A61K 31/18, A61K 31/197

(54) **Powder disinfectant for the teats of dairy animals**

(30) Priority: 14.12.2006 IT MI20062406
(71) Applicant: I.C.F. S.r.l., 26020 Plazzo Pignano (CR) (IT)
(72) Inventor: Venturini, Maurizio, 26100, CREMONA (IT)
(74) Representative: De Gregori, Antonella

(57) **Abstract**

The present invention relates to a disinfectant in powder form for the teats of dairy animals containing chloramines-T in synergic association with at least another active principle selected from EDTA, real aloe extract, xanthan rubber. The disinfectant of the present invention offers the advantage, with respect to the known disinfectants in the liquid state, of being produced in the form of a concentrated powder to be diluted in fresh water for use.

## Description

The present invention relates to a disinfectant for the teats of dairy animals.

The present invention derives from the field of disinfectants used in the treatment of the teats of dairy animals after milking.

Products are known, which exert a disinfecting action to protect the mammary system of dairy animals from the contamination of micro-organisms such as bacteria and fungi which are a recurrent source of infections.

The disinfectants currently used for this purpose are in the fluid state and are in the form of more or less viscous liquids.

As these disinfectants are sold in liquid form and packaged in large-sized containers, they require considerable space for their storage until their use.

One of the main objectives of the present invention consists in providing a disinfectant for the teats of dairy animals in powder form having softening, soothing, regenerating properties and a high filming capacity.

A further objective of the present invention is to provide a disinfectant for the teats of dairy animals in concentrated powder form which occupies a reduced space with respect to the preparations in which the chloramine is diluted in liquids.

According to a first aspect of the invention, a disinfectant is provided for veterinary use as specified in claim 1.

Further auxiliary characteristics of the invention are indicated in the subsequent claims.

The Applicant has found that the association of chloramine-T with at least one compound selected from EDTA, real aloe extract, xanthan rubber, allows a disinfecting composition to be obtained with high disinfecting, softening and soothing properties.

According to an aspect of the invention, the concentrated disinfectant in powder form with a high filming capacity should be used, after dilution, by immersion on the teats of the dairy animals immediately after milking. The reason for this is to protect the mammary system from possible external contaminating micro-organisms which are the recurrent cause of infections.

An embodiment of the invention envisages the association of chloramine-T, a chlorine-based compound having the formula 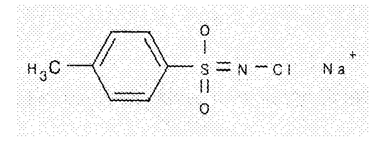 with dry real aloe extract, conveniently in an extraction ratio E/D of 1:100, which has high softening, soothing and cicatrizing properties and xanthan rubber, which provides a high filming capacity to the teats of animals.

Another embodiment envisages a disinfectant in which chloramine-T is synergized with EDTA or a derivative thereof, preferably disodium dihydrate salt.

According to another embodiment, chloramine-T is used as disinfecting substance with a wide range, in synergy with EDTA disodium dihydrate salt, dry extract of real aloe, having softening, soothing, regenerating properties and xanthan rubber, a natural thickening substance.

The disinfectant of the present invention offers the advantage, with respect to the other disinfectants currently used for this purpose in the liquid state, of being produced in solid form (powder), concentrated (to be diluted in fresh water at the moment of use) and in single-dose containers.

The product of the invention is typically formulated in concentrated powder form, to be diluted for use, envisaging, as an example, 50 g of product in 5 litres of water.

The disinfecting product of the invention should be conveniently used after milking by direct application on the teats of dairy animals, after dilution in water.

The disinfecting product of the invention can also contain additional active principles with a disinfecting and/or detergent action and other excipients and/or co-formulants acceptable from a veterinary point of view.

According to an embodiment of the invention, the disinfectant, object of the present invention, is packaged in 50 g single-dose containers.

For the use of the disinfecting product of the invention, the powder contained in the single-dose packages is conveniently poured into a can previously filled with 5 litres of fresh water. It is vigorously shaken until complete dispersion and is left to rest for 24 hours, after which it is preferably reshaken until homogeneity.

The viscous solution thus obtained is ready for use by immersion on the teats of dairy animals immediately after milking.

The following examples are provided for purely illustrative purposes of the present invention and should not be considered as limiting its protection scope, as defined by the enclosed claims.

### EXAMPLE 1

A preferred embodiment of the disinfecting product of the invention has the following composition per 100 g of product:

| | |
|---|---|
| 5 - 15 g | disodium dihydrate dihydrogenethylenediaminetetra-acetic acid |
| 5 - 15 g | dry real aloe extract |
| 15 - 35 g | chloramine-T |
| 50 - 75 g | xanthan rubber |

### EXAMPLE 2

In one of its more preferred embodiments, 100 g of the disinfecting composition comprise:

| | |
|---|---|
| 7 g | disodium dihydrate dihydrogenethylenediaminetetra-acetic acid |
| 10 g | dry real aloe extract |
| 25 g | chloramine-T |
| 58 g | xanthan rubber |

## Claims

1. A disinfectant for teats of dairy animals **characterized in that** it comprises chloramine-T associated with at least one compound selected from EDTA, real aloe extract, xanthan rubber and mixtures thereof.

2. The disinfectant according to claim 1, **characterized in that** it comprises a synergic association of chloramine-T and EDTA or a salt and/or derivative thereof.

3. The disinfecting product according to claim 2, **characterized in that** said EDTA salt is disodium dihydrate.

4. The disinfecting product according to any of claims 1-3, **characterized in that** it comprises chloramine-T, dry real aloe extract and xanthan rubber.

5. The disinfecting product according to any of claims 1-4, **characterized in that** it comprises chloramine-T, EDTA and/or its salts/derivatives, dry real aloe extract and xanthan rubber.

6. The disinfecting product according to any of claims 1-5, **characterized in that** it is in powder form.

7. The disinfecting product according to any of claims 1-6, **characterized in that** it is packaged in 50 g single-dose containers to be diluted at the moment of use in 5 1 of water.

8. The disinfecting product according to one or more of claims 1-7, **characterized in that** 100 g of said product contain:
| | |
|---|---|
| 5 - 15 g | disodium dihydrate dihydrogenethylenediaminetetra-acetic acid |
| 5 - 15 g | dry real aloe extract |
| 15 - 35 g | chloramine-T |
| 50 - 75 g | xanthan rubber |

9. The disinfecting product according to one or more of claims 1-7, **characterized in that** 100 g of said product contain:
| | |
|---|---|
| 7 g | disodium dihydrate dihydrogenethylenediaminetetra-acetic acid |
| 10 g | dry real aloe extract |
| 25 g | chloramine-T |
| 58 g | xanthan rubber |

10. Use of a product according to any of claims 1-9 for disinfection in the veterinary field.

11. Use of a product according to any of claims 1-9 for the disinfection of the teats of dairy animals.

12. Use according to claim 10 or 11, wherein said product is in powder form to be diluted extemporaneously in water.

13. Use according to claim 11 or 12 comprising the dilution of said disinfecting product in water and application on the teats of dairy animals after milking.
